# EUROPEAN PATENT SPECIFICATION

(11) **EP 1 282 392 B1**
(45) Date of publication and mention of the grant of the patent: **09.02.2005**
(21) Application number: 01951477.7
(22) Date of filing: 03.05.2001
(51) Int. Cl.: A61K 7/16

(54) **ORAL COMPOSITION**
ORALE ZUSAMMENSETZUNG
COMPOSITION ORALE

(30) Priority: 09.05.2000 EP 00303875
(43) Date of publication of application: 12.02.2003
(73) Proprietor: UNILEVER N.V., 3013 AL Rotterdam (NL); UNILEVER PLC, London EC4P 4BQ (GB)
(72) Inventor: DAVIES, Richard Hew, Unilever Italia SPA, 20083 Gaggiano (IT); KILCULLEN, Neil, Unilever Research Port Sunlight, Wirral, Merseyside L63 3JW (GB); WATERFIELD, Philip C., Unilever Res. Port Sunlight, Wirral, Merseyside L63 3JW (GB)
(74) Representative: Newbould, Frazer Anthony
(86) International application number: PCT/EP2001/004993
(87) International publication number: WO 2001/085114

(56) References cited:
- WO-A-97/06774
- WO-A-97/46216
- WO-A-98/22079
- WO-A-99/07335
- WO-A-99/55297
- GB-A- 1 130 929

## Description

The present invention relates to an oral composition according to the preamble of claim 1.

Oral care compositions comprising more than one formulation component are known in the art. Typically, the two formulations are separated to enable ingredients to be used which are unstable in certain critical conditions or to enable mixing of two ingredients immediately before brushing. The formulations are usually co-dispensed to deliver all ingredients in a single composition.

An advantage in having co-dispensed formulations is that the optimal conditions for storing a particular ingredient can be tailored in one part of the formulation for certain ingredients while a another formulation can be more conventional and contain the other ingredients common to oral care. For example, wintergreen flavour is a preferred flavour for toothpaste all over the world. One of the major ingredients in wintergreen oil is methyl salicylate. However, methyl salicylate is unstable in alkaline conditions due to natural hydrolysis of the ester link to produce an unpleasantly tasting by-product. Thus, alkaline formulations, such as chalk or bicarbonate formulations, themselves very popular, cannot sensibly comprise methyl salicylate. Thus the methyl salicylate has to be formulated in a neutral formulation which may then be co-dispensed with an alkaline bicarbonate or chalk paste.

Oral compositions comprising several formulations, usually two, which are stored independently from one another and dispensed together so that ingredients mix immediately before brushing are common in the art and are successfully marketed all over the world. Often, these formulations individually comprise ingredients which would be unstable in the presence of each other. Examples of such compositions on the market include the Mentadent® double pump products which comprise independently stored peroxide and bicarbonate formulations. The bicarbonate reacts with the peroxide when the two are mixed, i.e. on dispensing, to form oxygen bubbles. Clearly the bicarbonate and peroxide could not be stored together in the same formulation so they are stored independently of one another in modified packaging.

The main disadvantage with the above type of double pump product is that the packaging is more expensive than for a conventional tube and clearly if the packaging costs can be reduced so can the cost to the consumer.

However, it is also known in the prior art to separate two formulations within a single tube by using a membrane, which may be chemical or mechanical. An example of chemical separation can be found in US 4 098 435 (Weyn) which discloses two formulations separated by a gel layer, preferably sodium carboxymethyl cellulose-glycerol-sorbitol-water gel.

An example of a mechanical layer can be found in the marketplace. The Sensitive Maximum Strength product marketed by Colgate® is a conventional tube comprising two independently stored products which are separated by a divider which runs from the crimp end of the tube to the dispensing orifice. Despite this being marketed in a single tube form it still involves complicated and, therefore, expensive packaging to effect the dispensing of two independently stored formulations.

It is, therefore, an object of the invention to provide a composition comprising two formulations without the need for expensive packaging and without the need to separate them by means of a chemical or mechanical separating membrane.

We have surprisingly found that two formulations having different pH values can be stably stored adjacent one another without the pH of the two significantly balancing out within a period of six months from manufacture.

Accordingly, the invention provides an oral composition according to claim 1.

It is an essential feature of the invention that the first formulation and second formulation are stored adjacent one another. This means that they are stored so that they are in contact with one another as distinguished from the prior art in US 4 098 435 (Weyn).

A suitable device for storing a composition according to the invention is by way of the striped-stream toothpaste packaging disclosed in GB 813 514 (Unilever) and referred to in GB 1 130 929 (Unilever). Such a device is cheaper to manufacture than a double pump and is easier to use since it behaves as a single tube product.

Another suitable arrangement is where the two formulations are stored adjacent to one another and where the composition as a whole comprises each formulation in approximately equal proportions. For example, the different formulations could be twisted around each other so that they are dispensed as a striped paste in equal proportions. They may also be stored adjacent to one another but in a non-twisted arrangement where one formulation fills one side of the tube and another paste fills the other side of the tube.

It is also a feature of the invention that the pH of the first and second formulations differ by at least one pH unit. Since there is no limit as to the pH of either formulation the invention can be used to take advantage of any use of multiple, non-similar formulation technologies in the art which up until now have relied upon double pump packaging. However, it is preferred that one of the formulations has a pH in the range of from 7 to 10, more preferably from 8 to 9.5 while the other formulation has a pH in the range of from 6 to 9, more preferably from 6.5 to 8.

In a preferred embodiment the pH difference between the first and second formulations is at least one and a half pH units and especially at least two pH units.

Preferably, at least one of the formulations comprises a surfactant, preferably an anionic surfactant, at from 0.1 to 5% by weight of the composition. Preferably, the formulation comprises from 0.5 to 3% by weight of anionic surfactant. Suitable anionic surfactants include the alkali-metal alkyl sulphates such as sodium lauryl sulphate. In a particularly preferred embodiment, all the formulations in contact with each other contain surfactant, preferably, anionic surfactant.

Typically, at least one of the formulations will comprise from 1 to 99% of the oral composition. However, where the oral composition according to the invention is packaged in a device according to GB 813 514 one of the formulations, as a stripe paste, will comprise from 1 to 20% of the composition. However, clearly it is possible for any amount of the formulations to be present since they may even be included in equal amounts so as to produce a regular dual stream product.

It is also possible for any number of formulations to be present in the composition according to the invention in addition to the first and second formulation providing that the formulations maintain a pH difference of at least one pH unit.

In an alternative embodiment of the invention at least one of the formulations will comprise as abrasive chalk. Such a formulation will typically be formulated at an alkaline pH.

Furthermore, at least one of the formulations may comprise as abrasive silica. Such a formulation may be formulated at any pH commonly used in toothpaste technology. For example, if it comprises bicarbonate it will be of a more alkaline pH. In another embodiment it may be acidic in nature.

In an alternative aspect of the invention at least one of the formulations will comprise as flavour imparting agent wintergreen oil. Preferably such a formulation will have a substantially neutral pH. It is thus more likely that a silica paste having a substantially neutral pH will comprise any wintergreen imparting flavouring agent. It may also be possible for a wintergreen substitute, such as 2-hydroxypropiophenone, to be used as a further wintergreen flavour imparting agent and to be mixed with some methyl salicylate. In this way any increase in the pH of the silica composition when stored adjacent to another formulation of higher pH, e.g. a chalk formulation of pH ∼9, will still retain wintergreen flavour.

The oral composition according to the invention may contain any ingredients common in the art provided that they fulfil the requirements of claim 1.

Such ingedients include:
antimicrobial agents, e.g. Triclosan, chlorhexidine, copper-, zinc- and stannous salts such as zinc citrate, zinc sulphate, zinc glycinate, sodium zinc citrate and stannous pyrophosphate, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds, such as 2,2' methylenebis-(4-chloro-6-bromophenol);
anti-inflammatory agents such as ibuprofen, flurbiprofen, aspirin, indomethacin etc.;
anti-caries agents such as sodium- and stannous fluoride, aminefluorides, sodium monofluorophosphate, sodium trimetaphosphate and casein;
plaque buffers such as urea, calcium lactate, calcium glycerophosphate and strontium polyacrylates;
vitamins such as Vitamins A, C and E;
plant extracts;
desensitising agents, e.g. potassium citrate, potassium chloride, potassium tartrate, potassium bicarbonate, potassium oxalate, potassium nitrate and strontium salts;
anti-calculus agents, e.g. alkali-metal pyrophosphates, hypophosphite-containing polymers, organic phosphonates and phosphocitrates etc.;
biomolecules, e.g. bacteriocins, antibodies, enzymes, etc.;
flavours, e.g. peppermint and spearmint oils;
proteinaceous materials such as collagen;
preservatives; e.g. methyl paraben;
opacifying agents; e.g. titanium dioxide;
colouring agents; e.g. FD&C Blue No. 1, FD&C Yellow No. 5;
pH-adjusting agents; e.g. monosodium phosphate, trisodium phosphate, sodium hydroxide, sodium carbonate;
sweetening agents; e.g. sodium saccharin, aspartame;
pharmaceutically acceptable carriers, e.g. starch, sucrose, water or water/alcohol systems etc.;
surfactants, such as anionic, nonionic, cationic and zwitterionic or amphoteric surfactants;
particulate abrasive materials such as silicas, aluminas, calcium carbonates, dicalciumphosphates, calcium pyrophosphates, hydroxyapatites, trimetaphosphates, insoluble hexametaphosphates and so on, including agglomerated particulate abrasive materials, usually in amounts between 3 and 60% by weight of the oral care composition.
humectants such as glycerol, sorbitol, propyleneglycol, xylitol, lactitol etc.;
binders and thickeners such as sodium carboxymethylcellulose, xanthan gum, gum arabic etc. as well as synthetic polymers such as polyacrylates and carboxyvinyl polymers such as Carbopol®;
polymeric compounds which can enhance the delivery of active ingredients such as antimicrobial agents can also be included. Examples of such polymers are copolymers of polyvinylmethylether with maleic anhydride and other similar delivery enhancing polymers, e.g. those described in DE-A-3,942,643 (Colgate);
buffers and salts to buffer the pH and ionic strength of the oral care composition; and
other optional ingredients that may be included are e.g. bleaching agents such as peroxy compounds e.g. potassium peroxydiphosphate, effervescing systems such as sodium bicarbonate/citric acid systems, colour change systems, and so on.

Liposomes may also be used to improve delivery or stability of active ingredients.

The oral compositions may be in any flowable form common in the art, e.g. toothpaste, gel, mousse, cream, etc. and may also be formulated into systems for use in dual-compartment type dispensers.

Particular embodiments of the invention will now be illustrated in the following non-limiting examples:

### EXAMPLE

The following formulations were made according to standard methods and put into a stripe-packaging according to GB 813 514. The chalk phase (strand) represented 5% by weight of the composition and the silica phase (stripe) represented 95% by weight of the composition.

| **Chalk Phase** | |
|---|---|
| Ingredient | % (w/w) |
| Calcium carbonate | 35.0 |
| Sorbitol (70%) | 30.0 |
| Sodium lauryl sulphate | 2.5 |
| Silica | 2.0 |
| Sodium carboxymethyl cellulose | 0.9 |
| Titanium dioxide | 0.5 |
| Sodium monofluorophosphate | 0.8 |
| Trisodium ortho phosphate | 0.5 |
| Flavour | 0.75 |
| Sodium saccharin | 0.25 |
| Formalin | 0.1 |
| Water | 26.7 |

| **Silica Phase** | |
|---|---|
| Ingredient | % (w/w) |
| Silica | 20.0 |
| Sorbitol (70%) | 45.0 |
| Sodium lauryl sulphate | 1.5 |
| Sodium carboxymethyl cellulose | 0.9 |
| Polyethylene glycol 1500 | 5.0 |
| Sodium monofluorophosphate | 0.8 |
| Methyl salicylate | 7.0 |
| Colour | 0.5 |
| Formalin | 0.1 |
| Water | 19.2 |

The pH of the chalk phase is maintained at 9.5, while the pH of the silica phase is 6.

### EXAMPLE 2

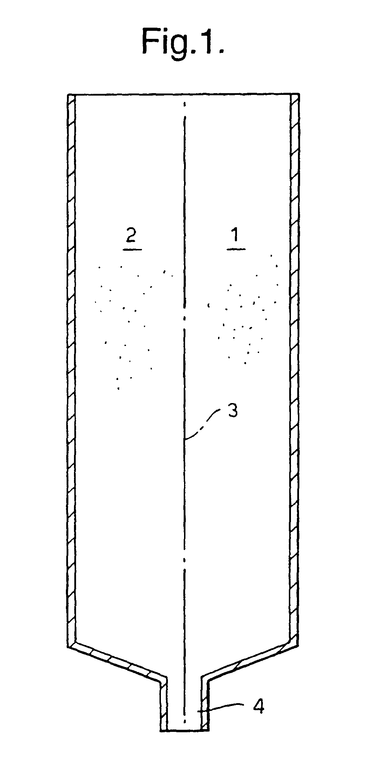

An oral composition comprising a chalk paste and a silica paste as described in Example 1 was made comprising 50% chalk paste and 50% silica paste. Each paste stored adjacent to the other in a single compartment tube as shown in figure 1.

Figure 1 shows a tube comprising an oral composition according to the invention. A chalk formulation (1) and a silica formulation (2) are stored adjacent to one another and meet at a central axis (3) along the middle of the tube. They are dispensed simultaneously through the dispensing orifice (4).

## Claims

1. Oral composition comprising a first formulation stored in contact with a second formulation for simultaneous dispensing, **characterised in that** the pH the first formulation differs from the pH of the second formulation by at least one pH unit.

2. Oral composition according to claim 1, **characterised in that** the pH of the first formulation differs from the pH of the second formulation by at least one and a half pH units.

3. Oral composition according to claim 1 or 2, **characterised in that** the pH of the first formulation differs from the pH of the second formulation by at least two pH units.

4. Oral composition according to any preceding claim, **characterised in that** at least one of the formulations comprises an anionic surfactant.

5. Oral composition according to any preceding claim, **characterised in that** the first formulation comprises from 1 to 99% by weight of the oral composition.

6. Oral composition according to any preceding claim, **characterised in that** at least one of the formulations comprises as abrasive chalk.

7. Oral composition according to any preceding claim, **characterised in that** at least one of the formulations comprises as abrasive silica.

8. Oral composition according to any preceding claim, **characterised in that** at least one of the formulations comprises as flavour imparting ingredient wintergreen oil.

## Patentansprüche

1. Orale Zusammensetzung, umfassend eine erste Formulierung, die in Kontakt mit einer zweiten Formulierung für das gleichzeitige Spenden gelagert wird, **dadurch gekennzeichnet, daß** sich der pH der ersten Formulierung vom pH der zweiten Formulierung um zumindest eine pH-Einheit unterscheidet.

2. Orale Zusammensetzung nach Anspruch 1, **dadurch gekennzeichnet, daß** sich der pH der ersten Formulierung vom pH der zweiten Formulierung um zumindest eineinhalb pH-Einheiten unterscheidet.

3. Orale Zusammensetzung nach Anspruch 1 oder 2, **dadurch gekennzeichnet, daß** sich der pH der ersten Formulierung vom pH der zweiten Formulierung um zumindest zwei pH-Einheiten unterscheidet.

4. Orale Zusammensetzung nach einem der vorhergehenden Ansprüche, **dadurch gekennzeichnet, daß** zumindest eine der Formulierungen ein anionisches oberflächenaktives Mittel umfaßt.

5. Orale Zusammensetzung nach einem der vorhergehenden Ansprüche, **dadurch gekennzeichnet, daß** die erste Formulierung 1 bis 99 Gew.-% der oralen Zusammensetzung umfaßt.

6. Orale Zusammensetzung nach einem der vorhergehenden Ansprüche, **dadurch gekennzeichnet, daß** zumindest eine der Formulierungen als Abriebmittel Calciumcarbonat umfaßt.

7. Orale Zusammensetzung nach einem der vorhergehenden Ansprüche, **dadurch gekennzeichnet, daß** zumindest eine der Formulierungen als Abriebmittel Siliciumdioxid umfaßt.

8. Orale Zusammensetzung nach einem der vorhergehenden Ansprüche, **dadurch gekennzeichnet, daß** zumindest eine der Formulierungen als Geschmack-verleihendes Mittel Wintergrün-Öl umfaßt.

## Revendications

1. Composition orale comprenant une première préparation, stockée en contact avec une seconde préparation destinée à l'administration simultanée, **caractérisée en ce que** le pH de la première préparation diffère du pH de la seconde préparation d'au moins une unité de pH.

2. Composition orale selon la revendication 1, **caractérisée en ce que** le pH de la première préparation diffère du pH de la seconde préparation d'au moins une unité et demie de pH.

3. Composition orale selon la revendication 1 ou 2, **caractérisée en ce que** le pH de la première préparation diffère du pH de la seconde préparation d'au moins deux unités de pH.

4. Composition orale selon l'une quelconque des revendications précédentes, **caractérisée en ce qu'**au moins l'une des préparations comprend un tensioactif anionique.

5. Composition orale selon l'une quelconque des revendications précédentes, **caractérisée en ce que** la première préparation comprend de 1 à 99% en poids de la composition orale.

6. Composition orale selon l'une quelconque des revendications précédentes, **caractérisée en ce qu'**au moins l'une des préparations comprend comme abrasif de la craie.

7. Composition orale selon l'une quelconque des revendications précédentes, **caractérisée en ce qu'**au moins l'une des préparations comprend comme abrasif de la silice.

8. Composition orale selon l'une quelconque des revendications précédentes, **caractérisée en ce qu'**au moins l'une des préparations comprend comme ingrédient conférant l'arôme de l'essence de wintergreen.
